# EUROPEAN PATENT APPLICATION

(11) **EP 3 236 250 A1**
(43) Date of publication of application: **25.10.2017**
(21) Application number: 15870438.7
(22) Date of filing: 16.11.2015
(51) Int. Cl.: G01N 27/417

(54) **SENSOR FOR SENSING HYDROGEN IN GASEOUS MEDIA**

(30) Priority: 15.12.2014 RU 2014150467
(71) Applicant: Joint Stock Company "Akme-Engineering", Moscow 115035 (RU)
(72) Inventor: MARTYNOV, Petr Nikiforovich, Obninsk Kaluzhskaya obl. 249030 (RU); CHERNOV, Michail Efimovich, Obninsk Kaluzhskaya obl. 249030 (RU); STOROZHENKO, Alexsey Nikolaevich, Obninsk Kaluzhskaya obl. 249034 (RU); SHELEMET'EV, Vasiliy Mikhaylovich, Obninsk Kaluzhskaya obl. 249030 (RU); SADOVNICHIY, Roman Petrovich, Obninsk Kaluzhskaya obl. 249030 (RU)
(74) Representative: Friese Goeden Patentanwälte PartGmbB
(86) International application number: PCT/RU2015/000791
(87) International publication number: WO 2016/099330

(57) **Abstract**

The device pertains to instrumentation technology and can be used in energy production, metallurgy, chemical industry to determine hydrogen concentration in gas media in a wide range of temperatures and pressures. The hydrogen detector in the gas medium comprises an operating element tightly fixed to the upper part of the detector housing by means of sealant. Additional leak-tightness is provided by a nut. The lower part of the detector is outlined by insulation that ensures a tight contact with a heater that provides operational temperature of the medium supplied to the waterproof membrane of the steam hydrogen compartment. Disturbance that is introduced by a measurement flow is transferred to the central core of the potential measuring unit through the measuring platinum electrode tightly fixed to the lower part of the ceramic sensing element tightly connected to the metal casing of the sensing element by the sealant. The standard electrode is located in the inner cavity of the ceramic sensing element. The external part of the ceramic sensing element bottom is covered with a layer of porous platinum electrode. The end of the potential measuring unit central core is brought out to the standard electrode. The technical result is the enhanced service life and reliability of the hydrogen detector operation in a wide range of working medium parameters by providing leak-tightness of the inner cavity of the ceramic sensing element and sustainability of medium temperature at the detector input. 3 figures

## Description

### Technical Field

The device pertains to instrumentation technology and can be used in energy production, metallurgy, chemical industry to determine hydrogen concentration in gas media in a wide range of temperatures and pressures.

### Background of the Invention

The electrochemical detector of hydrogen concentration in gas and fluid media is disclosed (refer to patent for the invention RU No. 2120624, IPC G01N27/417 *Electrochemical Detector of Hydrogen Concentration in Gas and Liquid Media,* published on 10/20/1998).

The detector comprises a housing tightly connected with solid electrolyte hydrogen detector by means of metal. The solid electrolyte oxygen detector consists of a ceramic insulator, closed in the lower part with a plug made of solid electrolyte, a porous platinum electrode applied on the external side of the plug, the liquid metal oxide standard electrode placed inside the plug, current lead thermocouple attached to the lid that covers the top of the ceramic insulator. A selective membrane shaped as a crimped cup is welded to the lower part of the housing. A tablet of the porous insulating oxide is installed between the selective membrane and the solid electrolyte plug.

The disadvantage of the said device is relatively low leak-tightness of the inner cavity of the ceramic sensing element that occurs due to oxygen inleakage through the gap between the potential measuring unit and the central core that results in oxidation of the reference electrode and decrease in service life of the device and reliability of its operation.

The electrochemical detector of hydrogen concentration in fluids and gases is disclosed (I.G. Dmitriev, V.L. Orlov, B.A. Shmatko. Electrochemical Hydrogen Detector in Fluids and Gases // The collection of abstracts of Teplofizika-91 (Thermophysics-91) Intersectoral Conference, Obninsk, 1993. p. 134-136).

The detector comprises an electrochemical oxygen cell based on solid electrolyte made of stabilized zirconium dioxide, a liquid-metal reference electrode of Bi+Bi203 mixture, a measuring platinum electrode, which is placed in a sealed chamber filled with water vapor.

The disadvantages of the known technical solution are:
- relatively low reliability and short service life of the device due to configuration complexity of the detector;
- relatively low thermal durability and corrosion resistance of the solid electrolyte oxygen detector to water vapors;
- relatively long response time and lack of sensitivity due to stabilization complexity of partial pressure of water vapor in the measuring chamber;
- relatively low accuracy of hydrogen concentration measurement, which is caused by difficulty of maintaining stability of temperature and pipes.

A hydrogen detector for gas and fluid media is technically the closest to the claimed device (refer to patent for invention RU 2379672 IPC G01N27/417 *Hydrogen Detector for Gas and Liquid Media,* published on 1/20/2008).

The hydrogen detector comprises a selective membrane, porous electrically insulating ceramics and a housing with a potential measuring unit inside, a ceramic sensing element made of solid electrolyte with a standard electrode in its cavity, a porous platinum electrode, applied to the external layer of the ceramic sensing element, silica fabric, joining material, a plug with a hole that covers the cross section of the cavity of the ceramic sensing element, a sealed lead-in tightly installed inside the housing above the ceramic sensing element, a doubly-clad cable potential measuring device that passes through the central hole of the sealed lead-in, a cylindrical bushing. The cavity of the housing between the sealed lead-in and the ceramic sensing element is leak-tight. The ceramic sensing element is designed as a cylinder interlinked with a part of the sphere, located in the lower part of the cylinder. The upper part of the external cylindrical surface of the ceramic sensing element is tightly connected to the inner side surface of the case by means of the joining material. The reference electrode is located in the cavity between the inner surface of the ceramic sensing element and the surface of the plug and occupies at least a part of the cavity. The external spherical part of the ceramic sensing element is covered with porous platinum electrode. The end of the central core of the potential measuring unit directed to the ceramic sensing element is brought out through the hole in the plug to the reference electrode. It enables an electric contact between the reference electrode and the lower part of the central core of the potential measuring unit. A part of the ceramic sensing element protrudes beyond the housing. The bushing shaped as a tube is connected to the lower part of the housing from the protruding part of the ceramic sensing element. The lower end of the bushing has a bottom with a center hole to which a selective membrane made of at least one tube is attached. The lower free end of the selective membrane is tightly closed with a plug. The cavity limited by the inner surface of the bushing, joining material, external part of the ceramic sensing element protruding beyond the housing and the inner surface of the selective membrane is leak-tight. The inner cavity of the bushing between the protruding part of the ceramic sensing element and the bushing bottom is filled with silica fabric. The porous electro-insulating ceramics designed as a cylinder is located with an annular gap to the inner surface of the selective membrane.

The disadvantage of the known device is relatively low leak-tightness of the inner cavity of the ceramic sensing element that may result in inleakages of oxygen to the inner cavity through the gap between the central core and the casing of the potential measuring unit and lead to oxidation of the reference electrode and decrease in service life of the device and reliability of its operation. Due to the absence of reliable leak-tightness of the upper part of the potential measuring unit, moisture may infiltrate into the insulating material of the doubly-clad cable, which may result in decrease of resistance of the central core and the cable sheath and, consequently, in the loss of the valid signal and deterioration of the detector reading.

### Invention Disclosure

The invention is aimed at increasing stability and reliability of hydrogen detector reading as well as its service life and reliability of its operation in a wide range of parameters in the gas medium.

### Technical Result

The technical result comprises enhanced measurement accuracy of the hydrogen detector reading by providing leak-tightness of the inner cavity of the ceramic sensing element and sustainability of stable operating temperature on the sensing part of the operating element ensured by constant reliable heating and thermal insulation that prevents heat leakage and oxidation of the detector reference electrode.

As a solution to the stated problem, we claim the detector design comprising a waterproof membrane made of at least one tube, provided with a measuring platinum electrode in the upper part and a housing with a potential measuring unit inside, a ceramic sensing element made of solid electrolyte. The ceramic sensing element cavity contains a reference electrode. The operating element is tightly fixed inside the housing above the sensing element. The potential measuring unit passes through the central hole and the lower part of the operating element, wherein the sensing element is designed as a cylinder interlinked with the bottom located in the lower part of the cylinder. The upper part of the potential measuring unit is leak-tight and contains a sealant with a tightly fixed nut. The external cylindrical surface of the sensing element is tightly connected to the inner side surface of the housing. The reference electrode is located inside the inner cavity of the sensing element. The end of the central core of the potential measuring unit is brought out into the reference electrode, wherein the electrical contact is provided between the reference electrode and the lower part of the central core of the potential measuring unit. The metal casing of the sensing element shaped as a tube is connected to the upper part of the sensing element by means of the sealant. The sealant is a glass-ceramic consisting of silicon oxide (SiO₂) - 45÷55 weight %, aluminum oxide (Al₂O₃) - 4÷6 weight %, boric oxide (B₂O₃) - 18÷22 weight %, titanium oxide (TiO₂) - 9÷12 weight %, sodium oxide (Na₂O) - 12÷15 weight %, potassium oxide (K₂O) - 1÷2 weight % and magnesium oxide (MgO) - 2÷3 weight %.

The sealant fills the ring-shaped cavity between the inner surface of the metal casing wall of the sensing element and the upper bushing and the external surface of the ceramic sensing element.

The detector is distinctive in that it is equipped with an additional thermally-insulated heater that serves for heating and sustaining stable operating temperature on the sensing part of the operating element; it also includes a steam hydrogen compartment consisting of a nickel case and a thin-walled waterproof membrane made of a thin-walled nickel tube that is welded to the sensing element casing, parts of which are made of corrosion resistant steel. The detector design allows for increasing stability and reliability of the hydrogen detector reading, as well as its service life and reliability of its operation in a wide range of parameters of the working medium.

### Brief Description of the Drawings

The invention is illustrated with drawings.

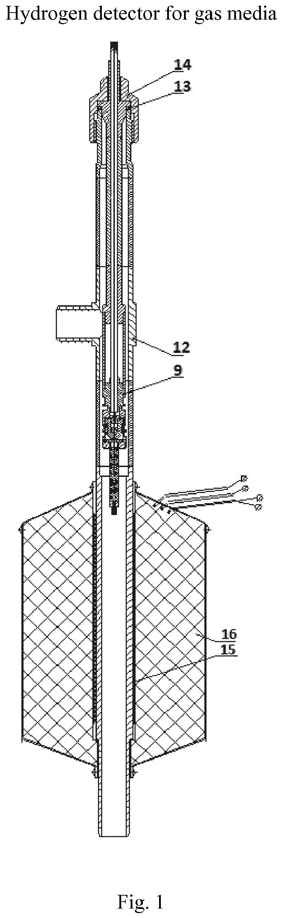

Fig.1 is a general view of the longitudinal axial cross-section of the detector.

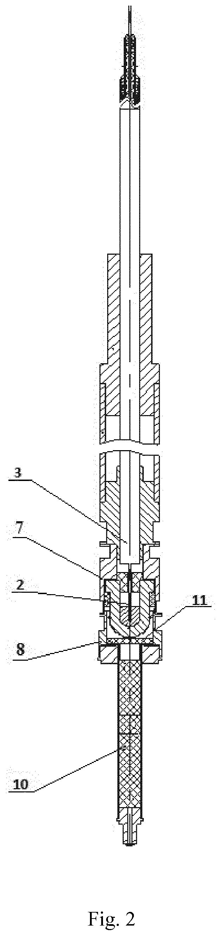

Fig.2 is a general view of the longitudinal axial cross-section of the detector operating element.

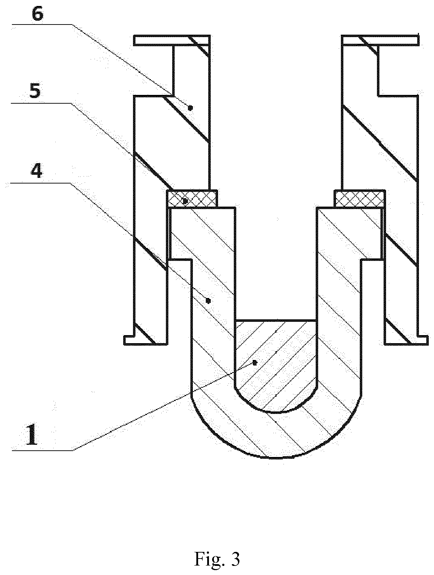

Fig.3 is a general view of the longitudinal axial cross-section of the detector sensing element.

### Embodiment of Invention

The hydrogen detector comprises a reference electrode 1 with immersed central core 2 of the potential measuring unit 3, located in the lower part of the ceramic sensing element 4 connected by means of glass-ceramic 5 with a metal case 6 of the sensing element 7 located inside the steam-water compartment 8. The represented items are included in the operating element 9 that has a bottom with a center hole to which a waterproof membrane 10 is attached. The membrane is made of at least one tube provided with a measuring platinum electrode 11 in its upper part. The operating element is located in the metal housing 12, leak-tightness of which is provided by sealant 3 and nut 14. A heater 15 with thermal insulation 16 serves for heating and sustaining stable operating temperature on the sensing part of the operating element.

The thermal insulation 16 fills the ring-shaped cavity between the inner surface of the heater wall 15 and the external surface of the housing of the detector that determines hydrogen concentration in gas 12.

The ceramic sensing element 4 is located in the lower part of the detector and shaped as a cylindrical part interlinked with the bottom.

The external cylindrical surface of the ceramic sensing element 4 is tightly connected to the inner side surface of the metal housing 12.

The reference electrode 1 is located in the inner cavity of the ceramic sensing element 4.

The housing 12 is designed as a tube connected with the metal casing of the sensing element 7.

The sealant 3 is a glass-ceramic consisting of silicon oxide (SiO₂)-50 weight %, aluminum oxide (Al₂O₃) - 5 weight %, boric oxide (B₂O₃) - 20 weight %, titanium oxide (TiO₂) - 10 weight %, sodium oxide (Na₂O) - 12 weight %, potassium oxide (K₂O) - 1 weight % and magnesium oxide (MgO) - 2 weight %.

The sealant is necessary to prevent ingress of oxygen from the air into the inner cavity of the detector and to avoid changes in the reference electrode properties.

The hydrogen detector applies the electrochemical method that allows to determine oxygen concentration by means of oxygen sensor made of solid oxide electrolyte. To measure hydrogen concentration in a gas medium, oxygen detectors are additionally equipped with a compartment of constant vapor pressure of water 8 and a waterproof membrane 10. Hydrogen contained the medium reversibly diffuses into the steam hydrogen compartment 8 through the membrane of the hydrogen detector to the measuring platinum electrode 11 changing the electromotive force of the detector. The electromotive force of the detector occurs due to differences in partial pressure of oxygen in the electrodes of the concentration cell. The scheme of the cell can be presented in the following way: Me - ES (an electrochemical sensor) - solid oxide electrolyte - AE (an actuating element) - H₂O, H₂ - H-membrane - the controlled medium.

The concentration cell includes the ceramic sensing element (CSE) 4 made of solid oxide electrolyte, the reference electrode (RE) 1 and the measuring platinum electrode (MPE) 11.

Partially stabilized zirconium dioxide (PSZD) based material was chosen to be used as solid oxide electrolyte. PSZD has high thermo-mechanical properties. Ionic conductivity within the temperature range of 300-400 °C may be up to 0.95 and is not less than 0.97 within the temperature range of 400-500 °C. Thermal impacts resistance exceeds 20 °C/ s.

*Bi - Bi*₂*O*₃ is used as a reference electrode 1 due to the stability of its thermodynamic properties.

Platinum-based porous composite coating is best suitable as a measuring (working) electrode 11 that serves as a catalyst for fast hydrogen oxidation on its surface. A special formula and method of application of this material on raw ceramic of the sensing element followed by annealing allows to produce high-porous working electrode of 30 µm thickness with good adhesive characteristics to ceramic.

The steam hydrogen compartment 8 is located in the cavity between the measuring platinum electrode 11 and the ceramic sensing element 4 and functions as a converter of hydrogen thermodynamic potential into oxidation potential of steam hydrogen mixture on the platinum electrode 11. Nickel is the most suitable material for the hydrogen membrane 10 due to its hydrogenous permeability and corrosion resistance in lead-bismuth eutectic.

### Industrial Applicability

The detector can be commercially manufactured. Moreover, its manufacturing does not require special equipment.

## Claims

1. The hydrogen detector for gas media comprises a waterproof membrane and a housing with a potential measuring unit inside, a ceramic sensing element made of solid electrolyte, the cavity of which contains a reference electrode, a porous platinum electrode, applied on the external layer of the ceramic sensing element, a sealed lead-in tightly fixed inside the housing above the ceramic sensing element, a potential measuring unit that passes through the central core of the sealed lead-in and the lower bushing, wherein the ceramic sensing element is designed as a cylinder interlinked with the bottom located in the lower part of the cylinder. The external cylindrical surface of the ceramic sensing element is tightly connected to the inner side surface of the housing. The standard electrode is located in the inner cavity of the ceramic sensing element. The external part of the bottom of the ceramic sensing element is covered with a layer of the porous platinum electrode. The end of the central core of the potential measuring unit is brought out to the standard electrode, thus the electrical contact is provided between the standard electrode and the lower part of the central core of the potential measuring unit. The lower bushing designed as a tube is connected to the lower part of the housing on the side of the ceramic sensing element. The lower end of the lower bushing has a bottom with a center hole with an attached selective membrane made of at least one tube. The lower free end of the selective membrane is tightly closed with a plug. The cavity limited by the inner surface of the lower bushing, the external part of the bottom of the ceramic sensing element and the inner surfaces of the selective membrane and the plug is made leak-tight by means of glass-ceramic sealant. The detector is equipped with an additional thermally-insulated heater that serves for heating and sustaining stable operating temperature on the sensing part of the operating element.

2. A detector according to claim 1, wherein oxygen sensors are additionally equipped with a compartment of constant vapor pressure of water and a waterproof membrane to measure hydrogen concentration in a gas medium more effectively.

3. A detector according to claim 1, wherein an upper nut is installed in the upper part of the potential measuring unit and the ring-shaped cavity between the inner surface of the nut wall and the external surface of the potential measuring unit is filled with sealant.

4. A detector according to claim 1, wherein its steam hydrogen compartment consisting of a nickel case and a thin-walled waterproof membrane made of a thin-walled nickel tube is welded to the sensing element casing made of corrosion resistant steel.
